**Europäisches Patentamt**

**European Patent Office**

**Office européen des brevets**

(19)

(11) Publication number: **0 068 905**
**A1**

(12) **EUROPEAN PATENT APPLICATION**

(21) Application number: **82303453.3**

(22) Date of filing: **01.07.82**

(51) Int. Cl.³: **A 01 C 3/02**

(30) Priority: **01.07.81 GB 8120393**

(43) Date of publication of application: **05.01.83**
Bulletin 83/1

(84) Designated Contracting States: **AT BE CH DE FR GB IT LI LU NL SE**

(71) Applicant: **S.C.S. BIOTECHNOLOGY LIMITED, Elmsfield Chipping Norton, Oxford OX7 5XS (GB)**
Applicant: **FARM GAS LIMITED, Industrial Estate, Bishops Castle Shropshire (GB)**

(72) Inventor: **Murcott, Andrew James, Ridge Farm Linley, Bishops Castle Shropshire (GB)**

(74) Representative: **Allsop, John Rowland et al, c/o Edward Evans & Co. Chancery House 53-64 Chancery Lane, London, WC2A 1SD (GB)**

(54) A holding tank or container for fluids, particularly for animal excreta in an aerobic or anaerobic digester.

(57) The tank or container is formed of prefabricated sections (1, 4) fastened together to form an enclosure (E) for holding the slurry.

The enclosure (E) has a profile (C) the curvature of which increases with the depth (h) of liquid which can be contained therein, in order that stress on the wall of the tank or container is approximately constant. In this way ease and cheapness of construction is accomplished in that the thickness of the walls of the tank or container may be made constant, and need not be specially strengthened.

The prefabricated sections are of two types, end sections (1) of which when fastened together form a substantially cylindrical tank or container in plan view, or two said end sections (1) with an extension section (4) of square or rectangular shape in plan view, between the two end sections (1).

This arrangement further simplifies construction in that there are only two types of prefabricated sections used to construct the tank or container for a large range of sizes.

"A Holding Tank or Container for Fluids  Particularly
for Animal Excreta in an Aerobic or Anaerobic Digester"

## FIELD OF THE INVENTION

The present invention relates to a holding tank or
container for fluids, in particular a tank for holding the
working medium in the form of slurry, of an anaerobic or
aerobic digester.

## BACKGROUND OF THE INVENTION

Anaerobic or aerobic digestion involves the treatment of
organic wastes, for example animal manures, by bacterial
decomposition.  This decomposition process provides inter
alia a valuable energy source in the form of methane gas,
while at the same time reducing the pollution hazard
associated with the waste both from the standpoint of
content and smell.

The apparatus involved in presently known digesters of the
type referred to, for containing the working medium tends
to be structurally complcated and lacks the versatility
of operation which is a main requirement, for example, when
being operated on the farm.

## SUMMARY OF THE INVENTION

It is an object of the present invention to provide a
holding tank or container particularly for the working
slurry in a bacterial digester, which obviates the above-
mentioned problems.

According to the invention there is provided a holding
tank or container for animal excreta slurry in an
anaerobic or aerobic digester comprising prefabricated
sections fastened together to form an enclosure for
holding the slurry at a predetermined level and
characterised in that the profile of the enclosure is curved, the curvature increasing with the depth of liquid containable within the enclosure.

This design of tank profile allows for a constant thickness of material, ease of manufacture and optimisation of the use of materials.

The prefabricated sections may be of two types, end sections which when fastened together form in plan view a cylindrical tank or container, or such end sections between which is inserted one or more central sections rectangular or square shaped in cross-section.

The advantage of this type of tank or container is that there are only two types of prefabricated sections for a large range of tank sizes which considerably simplifies construction.

Preferably the wall of each section is a sandwich of glass reinforced polyester and polyurethane foam, the foam being bonded to each of the two layers of polyester.

This method of fabrication renders the tank strong and corrosion resistant and provides the insulation necessary for the tank when used in an anaerobic digestion system.

Other features and advantages of the invention will become apparent to those skilled in the art from the following description of a preferred embodiment of the invention.

BRIEF DESCRIPTION OF THE DRAWINGS

Embodiments of the invention will now be described by way of example with reference to the accompanying
drawings wherein:

Figure 1 is a plan view of a holding tank or container
for use in an anaerobic digester, in its basic form;

Figure 2 is a plan view of the holding tank or container
of Figure 1 provided with a single extension section;

Figure 3 is a cross-sectional view of a holding tank
or container according to an embodiment of the invention
showing the curved profile;

Figure 4 is a detail at A of the wall of the tank
or container of Figure 3;

Figure 5 is a detail at B of the wall of the container
of Figure 3 at B; and

Figure 6 shows three different positions in which
the holding tank or container can be installed relative
to ground  level.

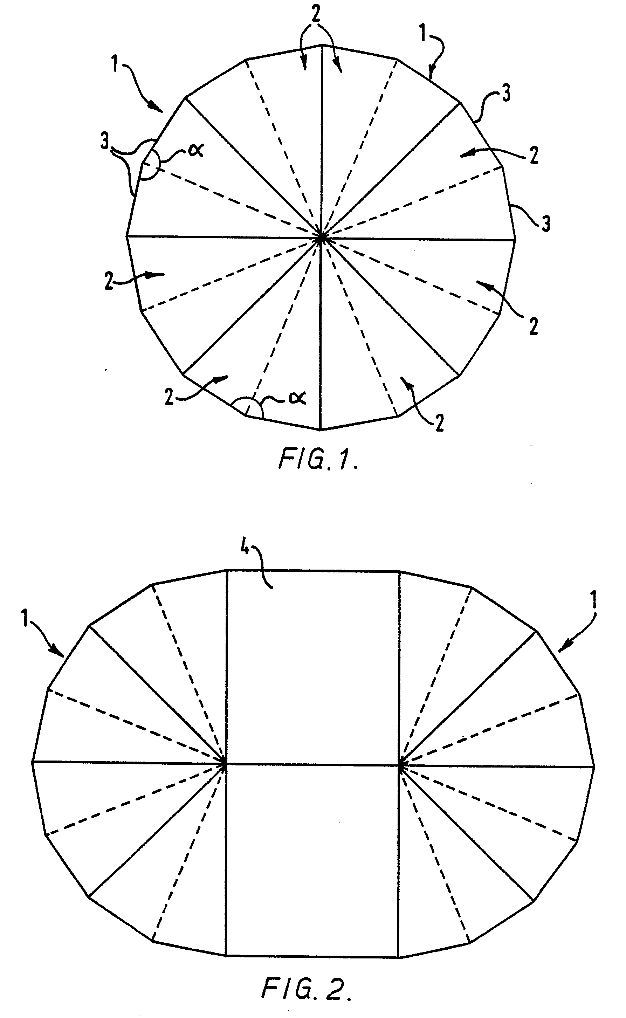

BEST MODES OF CARRYING OUT THE INVENTION
The basic form of the prefabricated holding tank or
container particularly for use in an anaerobic digester,
is shown in Figure 1.  It consists of a complete set
of end sections 1 which are bolted together to form
in plan view, a polygonal tank or container of substantially
circular configuration.

The end sections 1 each comprise four separate section
pieces 2 which are themselves fastened together, each
end section piece 2 being substantially wedge-shaped
and having two outer facing sides 3 angled at $\alpha$ degrees

to one another.  Thus each section 1 has eight outer facing sides in the form of a substantial semi-circle, making sixteen outer facing sides of the container or tank in total when the two end sections 1 are fastened together.

In Figure 2 the two end sections 1 of Figure 1 are separated by an extension section 4 which is rectangular in plan view as shown.

It will be appreciated that the holding tank or container can be further extended by adding further similar sections 4 between the two end sections 1 to form any desired size of tank or container.

Thus using only two types of prefabricated sections a large range of sizes of digester tank can be constructed.

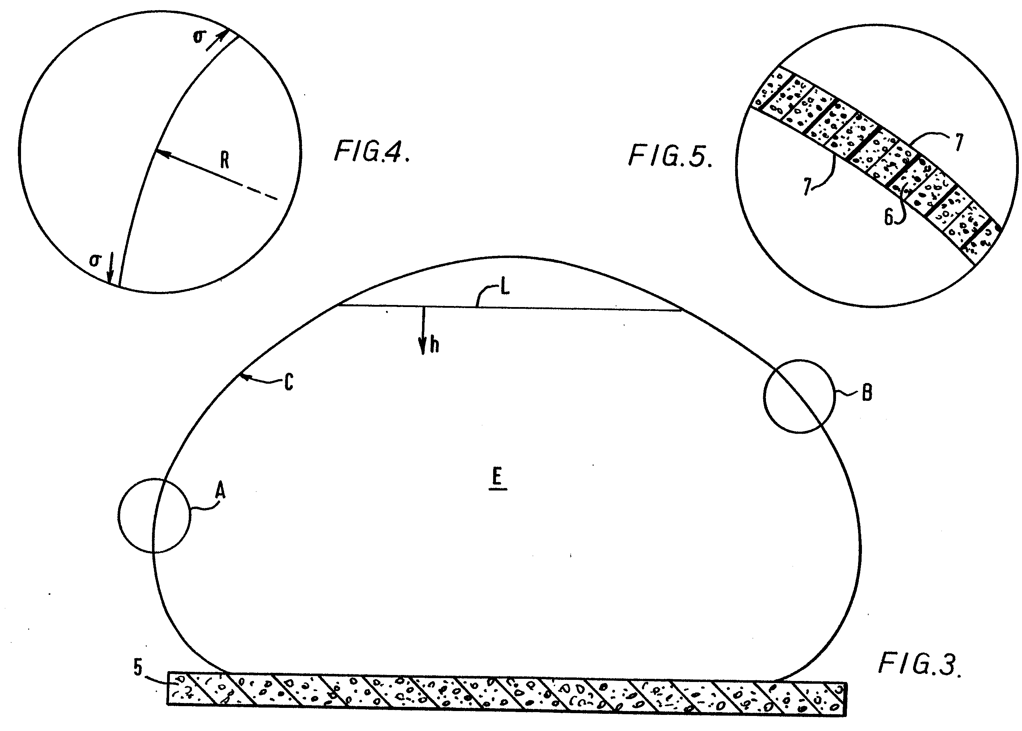

A typical tank or holding container according to the present invention is shown in cross-section in Figure 3 to illustrate its curved profile, the tank or container being mounted on a concrete base 5.  The tank or container forming an enclosure E, is shown filled with liquid up to a level L.

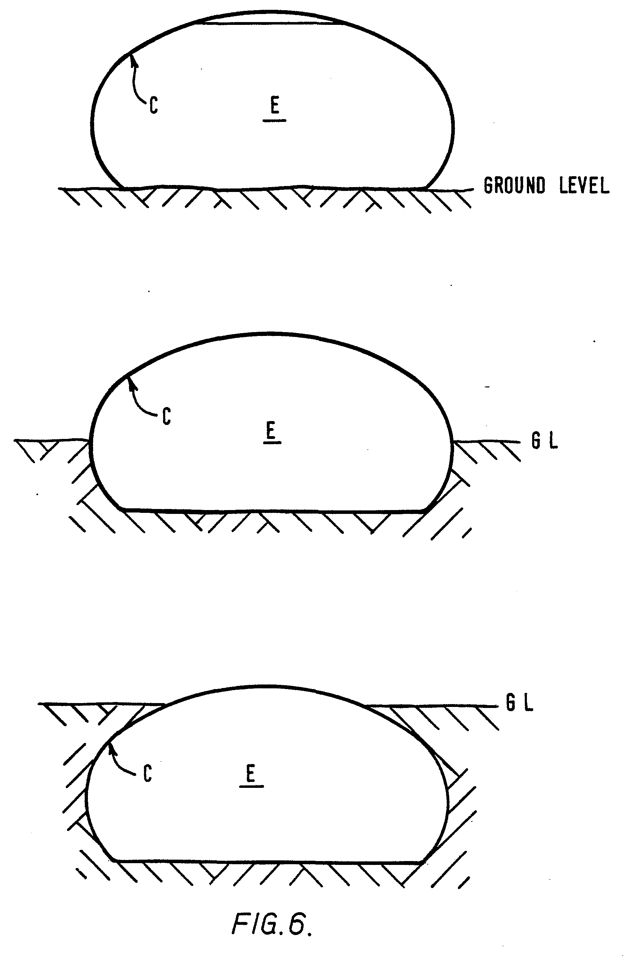

The detail A shown in Figure 4 of a portion of the curved profile (P) of the holding tank or container illustrates the relationship between the wall stress $\sigma$ and the radius of curvature R of the tank or container.

At any point on the wall of the tank the stress $\sigma$ is proportional to the head of liquid (i.e. it is proportional to the vertical distance down from the liquid surface h) and it is proportional to the radius of curvature of the tank profile at that point, R.

Thus $\sigma$ is proportional to hR.

Thus the profile of the tank or container shown in Figure 3 approximates to the condition that $\sigma$ ; and therefore hR is constant. This configuration allows for a constant thickness of material which results in ease of manufacture and optimum use of materials. The curvature of the tank wall profile C is equal to 1/R, and the condition that is hR is constant is satisfied if the curvature C is directly proportional to the depth of the liquid in the tank or container. Thus it can be seen that the curvature of the profile C increases with the depth of liquid L in order that the wall stress is approximately constant.

A further enlarged detail B of the wall of the tank or container shown in Figure 3 is shown in Figure 5. It can be seen to comprise a layer of polyurethane foam 6 sandwiched between two layers of glass reinforced polyester 7. This provides strength and insulation properties, the latter being a particular requirement for use in anaerobic digesters.

It must also be noted that each of the prefabricated sections, that is the four sections 2 in each end section 1, and the extension sections 4, may be provided with a manhole or similar openings. For example in one manhole may be mounted a vertical heat exchanger, in another a mechanical stirrer, in another a pressure relief valve and in another a gas outlet, and so on.

When used in an anaerobic digester the contents of the holding tank or container must be agitated. In the case of liquid slurry this agitation would be the recirculation of compressed methane gas drawn out of the digester, compressed, and then fed back in, normally at the bottom of the holding tank or container.

Thus employing the tank or container of the present invention the pipelines for the return of this compressed gas may be built into the prefabricated structure. In the case of solid manure or very thick slurry being fed into the digester, then in addition there would be a mechanical agitation by a propeller installed through one or more of the manhole covers in the centre sections as explained above.

The holding tank or container as above described is designed to be free-standing, partially buried in the ground, or completely buried, and this is illustrated in Figure 6. It will be appreciated that assembly on site can be carried out either by the customer himself or by contractors. in summary, the advantages of the construction as above described can be summarised as follows:

1.   There are only two types of prefabricated tank sections for a larage range of tank sizes.

2.   The fabrication method being of glass reinforced polyester with integral bonded foam, a strong corrosion resistant, and provides insulation.

3.   The profile of the tank or container allows for a constant thickness of material resulting in ease of manufacture and optimum usage of materials.

4.   The tank or container can be free-standing, partially buried, or completely buried.

5.   Since it can be buried it is possible to scrape or otherwise load into the tank or container which has particular applicability to anaerobic digestion on the farm when loading with high fibre content, for example straw-based farmyard manure.

6. The tank or container can be readily assembled on a particular site by the customer, which again has particular relevance to farmyard use.

Modifications and improvements of the above-described tank or container will be clear to those skilled in the art within the broad inventive scope provided by the present invention. Moreover it will be appreciated that the tank or container has applicability to fields of use other than in anaerobic digestion of waste material and is also suitable for the storage of processing of other substances.

## CLAIMS

1. A holding tank or container for animal excreta slurry in an anaerobic or aerobic digester comprising prefabricated sections (1,4) fastened together to form an enclosure (E) for holding the slurry at a predetermined level (L) and characterised that the profile (C) of the enclosure (E) is curved, the curvature increasing with the depth of liquid (h) containable within the enclosure (E).

2. A holding tank or container as claimed in Claim 1 wherein the curvature of the profile (C) is directly proportional to the depth of liquid (h), containable within the enclosure (E).

3. A holding tank or container as claimed in Claim 1 or 2 wherein the prefabricated sections comprise a pair of end sections (1) fastened together to form in plan view a substantially cylindrical tank or container.

4. A holding tank or container as claimed in Claim 3 wherein said end sections (1) comprise four separate wedge-shaped end section pieces (2) having two outer facing sides angled($\alpha$) to one another to provide eight outer facing sides (3) around the periphery of each said end section pieces (2) in the form of a substantial semi-circle.

5. A holding tank or container as claimed in Claim 4 wherein one or more extension sections (4) of rectangular or square shaped in plan view, is or are fastened between two said end sections (1).

6. A holding tank or container as claimed in Claim 5 wherein the wall of the end and extension sections (1) and (4) comprises a layer of polyurethane foam (6) sandwiched between the layers of glass reinforced polyester (7).

7. A holding tank or container for fluids such as animal excreta in a bacterial digester comprising prefabricated sections (1,4) assembled together to form an enclosure (E), characterised in that the sections are of two types, end sections (1) which when joined together form in cross-sectional plan view a substantially cylindrical tank or container, and extension sections (4) which are rectangular or square in cross-sectional plan view mounted between two said end sections (1), the radius of curvature of the enclosure (E) increasing with the depth (h) of liquid containable within the enclosure (E).

FIG.1.

FIG.2.

FIG.5.

FIG.4.

FIG.3.

GROUND LEVEL

G L

G L

FIG.6.

European Patent Office

# EUROPEAN SEARCH REPORT

Application number

EP 82 30 3453.3

| Category | Citation of document with indication, where appropriate, of relevant passages | Relevant to claim |
|---|---|---|
| | **DOCUMENTS CONSIDERED TO BE RELEVANT** | |
| A | US - A - 4 254 885 (J.L. FOUSS et al.) <br> * abstract; fig. 1 to 6 * <br> -- | 1 |
| A | FR - A1 - 2 292 415 (M. ISMAN et al.) <br> * page 2, lines 15 to 19; fig. 1 * <br> -- | 6 |
| A | DE - B2 - 2 756 455 (J. FEILHAUER GEB. WIESE) <br> ---- | |

**CLASSIFICATION OF THE APPLICATION (Int. Cl. 3)**

A 01 C    3/02

**TECHNICAL FIELDS SEARCHED (Int.Cl. 3)**

A 01 C    3/00

B 29 C    24/00

B 65 D    88/16

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 23-08-1982 | SCHOFER |

EPO Form 1503.1   06.78